# EUROPÄISCHE PATENTANMELDUNG

(11) **EP 0 548 739 A2**
(43) Veröffentlichungstag der Anmeldung: **30.06.1993**
(21) Anmeldenummer: 92121287.4
(22) Anmeldetag: 15.12.1992
(51) Int. Cl.: C07C 33/025, C07C 29/44

(54) **Verfahren zur Herstellung von Alkenolen**

(30) Priorität: 21.12.1991 DE 4142515
(71) Anmelder: BASF Aktiengesellschaft, D-67063 Ludwigshafen (DE)
(72) Erfinder: Klein, Ulrich, Dr., W-6703 Limburgerhof (DE); Buschmann, Ernst, Dr., W-6700 Ludwigshafen (DE); Mackenroth, Christiane, Dr., W-6702 Bad Duerkheim (DE)

(57) **Zusammenfassung**

Verfahren zur Herstellung von Alkenolen der Formel I

R¹-CH=CH(CH₂)ₙOH I

in der R¹ für Wasserstoff oder einen Kohlenwasserstoffrest steht und der Index n eine ganze Zahl von 3 bis 15 bedeutet,
a) durch Umsetzung eines Phosphoniumsalzes der Formel IIa

   R¹-CH₂-P⁺(C₆H₅)₃ X⁻ IIa

   in der X für Chlor, Brom und Jod steht, mit einem Aldehyd der Formel IIIa oder seinem Halbacetal der Formel IIIb 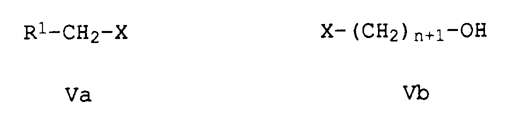 oder
b) durch Umsetzung eines Phosphoniumsalzes der Formel IIb

   (C₆H₅)₃P⁺(CH₂)ₙ₊₁OH X⁻ IIb

   mit einem Aldehyd der Formel IV

   R¹CHO IV

   in einem Lösungsmittel in Gegenwart einer Base, dadurch gekennzeichnet, daß man als Base das Alkalimetallsalz eines Alkohols und als Lösungsmittel einen Alkohol verwendet.

## Beschreibung

Die vorliegende Erfindung betrifft ein Verfahren zur Herstellung von Alkenolen der Formel I

R¹-CH=CH(CH₂)ₙOH (I)

in der R¹ für Wasserstoff oder einen Kohlenwasserstoffrest steht und der Index n eine ganze Zahl von 3 bis 15 bedeutet,
a) durch Umsetzung eines Phosphoniumsalzes der Formel IIa

   R¹-CH₂-P⁺(C₆H₅)₃ X⁻ IIa

   in der X fr Chlor, Brom und Jod steht, mit einem Aldehyd der Formel IIIa oder seinem Halbacetal der Formel IIIb 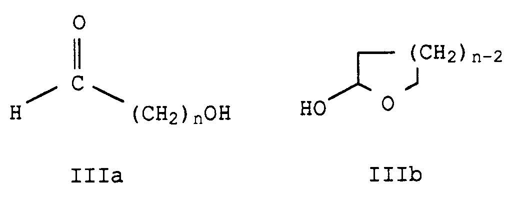 oder
b) durch Umsetzung eines Phosphoniumsalzes der Formel IIb

   (C₆H₅)₃P⁺(CH₂)ₙ₊₁OH X⁻ IIb

   mit einem Aldehyd der Formel IV

   R¹CHO IV

   in einem Lösungsmittel in Gegenwart einer Base, welches dadurch gekennzeichnet ist, daß man als Base das Alkalimetallsalz eines Alkohols und als Lösungsmittel einen Alkohol verwendet.

Die vorliegende Erfindung betrifft des weiteren ein Verfahren zur Herstellung von Alkenolen ausgehend von der Umsetzung eines Alkylhalogenids der allgemeinen Formel Va bzw. Vb 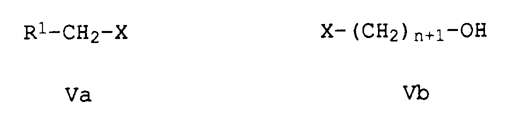
mit Triphenylphosphin bzw. ausgehend von der Umsetzung eines Alkohols der allgemeinen Formel VI

R¹-CH₂-OH VI

mit einer Halogenwasserstoffsäure zum entsprechenden Alkylhalogenid der Formel Va.

Außerdem betrifft die Erfindung Verfahren zur Herstellung von 5-Decen-1-ol ausgehend von n-Pentanol, Verfahren zur Herstellung von 4-Tridecen-1-ol ausgehend von n-Nonanol, Verfahren zur Herstellung von 8-Dodecen-1-ol ausgehend von 8-Bromoctan-1-ol und Verfahren zur Herstellung von 9-Dodecen-1-ol ausgehend von 9-Bromoctan-1-ol.

Alkenole der Formel I haben Bedeutung als Pheromone oder Synthesevorstufen für Pheromone. (Strukturübersicht siehe: List of sexpheromones of Lepidoptera and related attractants, H. Arn., M. Toth und E. Priesner, OILB, 1986). Sie werden als Lockstoffe in Insektenfallen zur Befallserkennung eingesetzt. Dafür werden weltweit jedoch nur Kilogramm-Mengen gebraucht, die nach Labormethoden hergestellt werden können.

Die Entwicklung der Methode der Paarungsstörung mit Aufwandmengen von 10 bis 1000 g Pheromon pro Hektar und Jahr führt zu einem wachsenden Bedarf an Pheromonwirkstoffen. (Eine Übersicht über die Verwendung von Pheromonen im Rahmen der Paarunsstörungsmethode geben A.K. Minks und R.T. Cardé in Entomol. exp. appl. 49, 25 - 36 (1988)). Die für das Labor konzipierten Synthesemethoden (siehe z. B. K. Mori, The Synthesis of insect pheromones in: The total synthesis of naturalproducts, Band 4, S. 1ff., Wiley-Interscience, New York, 1981; C.A. Henrick, Tetrahedron 33, 1845 - 1889 (1977)) müssen deshalb so verändert werden, daß sie großtechnisch zur Synthese der Verbindungen im Tonnenmaßstab angewendet werden können.

Alkenole werden industriell bisher vielstufig über Alkinvorstufen hergestellt (z.B. EP-A 32,396). Ausgangsverbindungen sind Acetylen oder Alkine, deren Umsetzung ein gewisses Sicherheitsrisiko bedeutet, ebenso wie die bei diesen Synthesen mehrfachnotwendigen Metallierungsschritte.

Außerdem ist es bekannt, Alkenole mit Hilfe der Wittig-Reaktion herzustellen. Derartige Synthesen verlaufen über nicht stabilisierte Phosphonium-Ylide.

Wittig-Reaktionen von nicht stabilisierten Phosphoniumyliden wie IIa und IIb werden bisher in Lösungsmitteln wie Tetrahydrofuran, Dioxan, Dimethylformamid, Hexamethylphosphorsäuretriamid, Dimethylsulfoxid oder ähnlichen Lösungsmitteln durchgeführt (s.Houben-Weyl, Methoden der Organischen Chemie, Thieme 1972, Band V 1b, S. 383ff.). Diese Lösungsmittel haben den Nachteil, daß sie bei der wässrigen Aufarbeitung der Reaktionsansätze auf Grund ihrer Hydrophilie in der wäßrigen Phase gelöst bleiben, so daß die Entsorgung aufwendig und teuer wird. Solche Lösungsmittel scheiden daher aus ökonomischen und ökologischen Gründen für die Herstellung im industriellen Maßstab aus.

Alternativ können auch Kohlenwasserstoffe wie Benzol, Toluol, Xylole, oder Hexan eingesetzt werden (siehe C. Szantay et al. Acta Chim. Hung. 125, 797 (1988)). Diese ermöglichen eine wäßrige Aufarbeitung der Reaktionsansätze, die zu weniger belasteten Abwässern führt. Nachteil ist jedoch, daß die Reaktion sehr verdünnt gefahren werden muß, damit die Phosphoniumsalzsuspension rührbar bleibt. Die Raum/Zeit-Ausbeuten sind sehr gering.

Als Basen werden bisher eingesetzt: n-Butyllithium, Kalium in HMPT, Natriumhydrid, Natriumhexamethyldisilazid, aber auch besser handhabbare und preiswertere Basen wie Kalium-tert.-butylat, Natriummethylat, Natriumethylat, Kaliummethylat und Kaliumethylat, die ebenfalls zu guten Ausbeuten in unpolaren Lösungsmitteln führen. Wittig-Reaktionen mit stabilisierten Yliden, wie z. B.

Ph₃P⁺CH₂CO₂Et IIb

verlaufen in polaren protischen Lösungsmitteln wie z.B. in Alkoholen mit guten Ausbeuten und werden deshalb auch industriell breit eingesetzt.

Nicht stabilisierte Ylide, wie sie zur Herstellung der Alkenole I erforderlich sind, liefern nach dem gegenwärtigen Wissensstand bei der Umsetzung in Alkoholen schlechte Ausbeuten, so daß beim Einsatz von Alkoholatbasen sogar empfohlen wird, die dabei entstehende geringe Alkoholmenge sorgfältig vor der Weiterreaktion zu entfernen (C. Szantay, Acta Chim. Hung. 125 (6), 807(1988)).

Aus den oben beschriebenen Gründen hat die Wittig-Reaktion mit nicht stabilisierten Yliden bisher kaum Eingang in die industrielle Praxis gefunden.

Es wurde nun gefunden, daß man Alkenole der Formel I besonders vorteilhaft auch großtechnisch dadurch herstellen kann, daß man entweder
a) ein Phosphoniumsalz der Formel IIa mit einem Aldehyd der Formel IIIa oder seinem Halbacetal der Formel IIIb oder
b) ein Phosphoniumsalz der Formel IIb mit einem Aldehyd der Formel IV
in einem Lösungsmittel in Gegenwart einer Base umsetzt, wobei man als Base das Alkalimetallsalz eines Alkohols und als Lösungsmittel einen Alkohol verwendet. 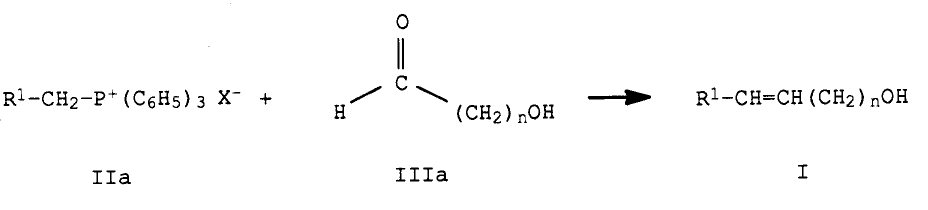
bzw. 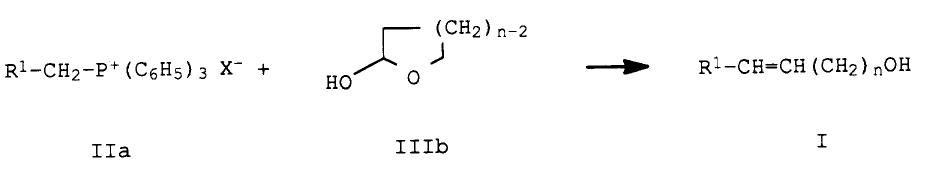
oder 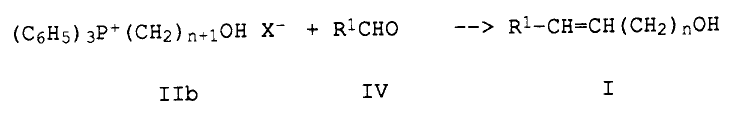
In den Formeln IIa und IIb steht X für Chlor, Brom und Jod, bevorzugt Brom.

n in den Formeln I, IIb und IIIa bzw. IIIb steht für eine ganze Zahl von 3 bis 15.

Nach den bisherigen Erkenntnissen hat der Wert der Zahl n lediglich bei der Umsetzung von Halbacetalen der Formel IIIb in sofern einen Einfluß, als 5- bzw. 6-gliedrige cyclische Halbacetale (n = 3 oder 4) infolge ihrer Stabilität bevorzugt werden.

Der Rest R¹ in den Formeln IIa bzw. IV steht für Wasserstoff oder einen Kohlenwasserstoffrest.

Als Kohlenwasserstoffreste kommen
Alkylgruppen mit 1 bis 20 Kohlenstoffatomen, bevorzugt C₁-C₁₅-Alkylgruppen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, sowie die entsprechenden unverzweigten oder verzweigten Heptyl-, Octyl-, Nonyl-, Decyl-, Undecyl-, Dodecyl-, Tridecyl- und Pentadecylreste.

Alkenylgruppen mit 2 bis 20 Kohlenstoffatomen, bevorzugt C₂-C₁₅-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, sowie die entsprechenden unverzweigten und verzweigten Heptenyl-, Octenyl-, Nonenyl-, Decenyl-, Undecenyl-, Dodecenyl-, Tridecenyl-, Tetradecenyl- und Pentadecenylreste.

Alkinylgruppen mit 2 bis 20 Kohlenstoffatomen, bevorzugt C₂-C₁₅-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, sowie die entsprechenden unverzweigten und verzweigten Heptinyl-, Octinyl-, Noninyl-, Decinyl-, Undecinyl-, Dodecinyl-, Tridecinyl-, Tetradecinyl- und Pentadecinylreste.

Cycloalkylgruppen mit 3 bis 17 Kohlenstoffatomen, bevorzugt C₃-C₈-Cycloalkyl wie Cyclopropyl, Cyclobutyl, Cyclopentyl, Cyclohexyl, Cycloheptyl und Cyclooctyl,
Cycloalkenylgruppen mit 5 bis 17 Kohlenstoffatomen, bevorzugt C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-4-enyl,
Cycloalkdienylgruppen mit 5 bis 12 Kohlenstoffatomen, bevorzugt C₅-C₈-Cycloalkdienyl wie Cyclopenta-1,3-dien-1-yl, Cyclopenta-1,3-dien-2-yl, Cyclopenta-1,3-dien-5-yl, Cyclohexa-1,3-dien-1-yl, Cyclohexa-1,3-dien-2-yl, Cyclohexa-1,3-dien-5-yl, Cyclohexa-1,4-dien-1-yl, Cyclohexa-1,4-dien-3-yl, Cyclohepta-1,3-dien-1-yl, Cyclohepta-1,3-dien-2-yl, Cyclohepta-1,3-dien-5-yl, Cyclohepta-1,3-dien-6-yl, Cyclohepta-1,4-dien-1-yl, Cyclohepta-1,4-dien-2-yl, Cyclohepta-1,4-dien-3-yl, Cyclohepta-1,4-dien-6-yl, Cycloocta-1,3-dien-1-yl, Cycloocta-1,3-dien-2-yl, Cycloocta-1,3-dien-5-yl, Cycloocta-1,3-dien-6-yl, Cycloocta-1,4-dien-1-yl, Cycloocta-1,4-dien-2-yl, Cycloocta-1,4-dien-3-yl, Cycloocta-1,4-dien-6-yl, Cycloocta-1,4-dien-7-yl, Cycloocta-1,4-dien-1-yl und Cycloocta-1,4-dien-3-yl, und
aromatische Systeme wie Phenyl und Naphthyl in Betracht.

Diese Kohlenwasserstoffreste können ihrerseits Substituenten tragen, sofern sich die Substituenten unter den Reaktionsbedingungen inert verhalten. Beispiele für derartige Substituenten sind Alkylgruppen mit ein bis sechs Kohlenstoffen (bei cyclischen Kohlenwasserstoffen), dementsprechende Alkoxy- oder Alkylthiogruppen, cyclische Gruppen wie vorstehend genannt, unsubstituierte oder substituierte aromatische Reste wie insbesondere Phenyl und Halogenatome wie vorstehend genannt, wobei diese Substituenten nach den bisherigen Erkenntnissen keinen Einfluß auf die Umsetzung haben.

Im Hinblick auf die Verwendung der Syntheseprodukte als Pheromone besonders bevorzugte Kohlenwasserstoffreste in der Bedeutung R¹ sind: Alkyl-, Alkenyl- und Alkinylreste mit 5 bis 15 Kohlenstoffen.

Die erfindungsgemäßen Umsetzungen werden im einzelnen wie folgt durchgeführt:
- A:: Umsetzung eines Phosphoniumsalzes der Formel IIa mit einem Aldehyd der Formel IIIa oder seinem Halbacetal der Formel IIIb 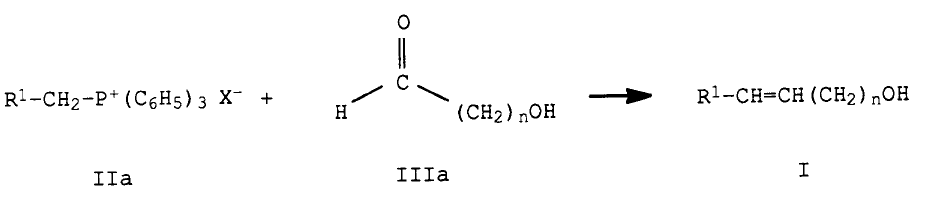 bzw. 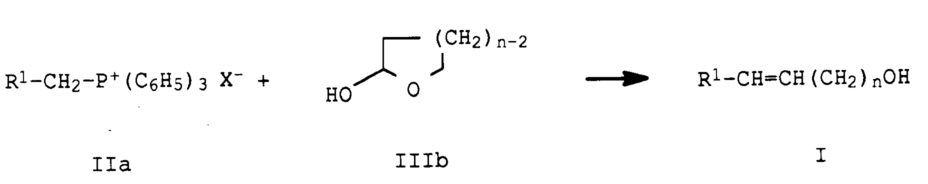
Im allgemeinen liegt die Reaktionstemperatur bei Wittig-Umsetzungen zwischen 0°C und der Siedetemperatur des verwendeten Lösungsmittels. Das in situ hergestellte Phosphorylid wird üblicherweise anschließend mit dem Aldehyd (siehe Houben-Weyl, Methoden der organischen Chemie, Thieme 1972, Band V, 1b, S. 383ff) umgesetzt.

Bei dem erfindungsgemäßen Verfahren liegt die Reaktionstemperatur im allgemeinen zwischen -50 und 150°C, bei Verwendung der Aldehyde IIIa bevorzugt zwischen 50 und 100°C, bei Verwendung der Aldehyde IV bevorzugt zwischen 0 und 50°C.

Erfindungsgemäß verwendet man für diese Umsetzung als Lösungsmittel einen Alkohol. Derartige Alkohole sind beispielsweise C₁-C₁₅-Alkohole, wobei der Verzweigungsgrad nur insofern eine Rolle spielt, als der verwendete Alkohol bei der Umsetzungstemperatur flüssig sein muß.

Als Basen dienen vorzugsweise niedermolekulare Alkalialkoholate wie Natriummethylat, Natriumethylat, Kaliummethylat, Kaliumethylat, Natrium-tert.-butylat, Kalium-tert.-butylat. Besonders günstig sind Alkalialkoholatlösungen im entsprechenden Alkohol, weil dies verfahrenstechnisch eine Vereinfachung darstellt. Man verwendet hier z. B. Natrium- oder Kaliummethanolat in Methanol oder Natrium- oder Kaliumethanolat in Ethanol. Es ist vorteilhaft, das Lösungsmittel des Alkoholats (z.B. Methanol oder Ethanol) nach der Basenzugabe aus dem Reaktionsansatz abzudestillierten. Das Reaktionsvolumen bleibt dadurch klein. Der Alkohol kann wiederverwendet werden und stört nicht die anschließende wäßrige Aufarbeitung.

Die Basen werden im allgemeinen mindestens in äquimolaren Mengen eingesetzt, üblicherweise verwendet man sie aber in einem Überschuß von 10 bis 150%, vorzugsweise 50 bis 100%.

Die Edukte werden im allgemeinen in äquimolaren Mengen miteinander umgesetzt. Es kann für die Ausbeute vorteilhaft sein, den Aldehyd in einem Überschuß von 10 bis 20% bezogen auf das Phosphoniumsalz einzusetzen.

Die Reaktionsgemische werden zunächst mit Wasser versetzt und anschließend in üblicher Weise aufgearbeitet, z.B. durch Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.
- B:: Umsetzung eins Phosphoniumsalz der Formel IIb mit einem Aldehyd der Formel IV

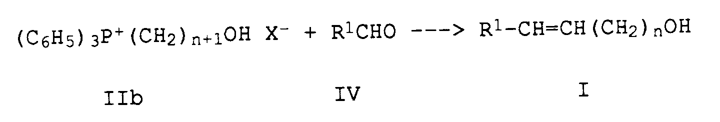

Diese Umsetzung wird im allgemeinen und im besonderen unter den in Verfahren A beschrieben Bedingungen durchgeführt.

Die für die Umsetzungen A und B benötigten Ausgangsstoffe lassen sich in besonders einfacher Weise in situ im Reaktionsmedium vorab herstellen.

Phosphoniumsalze der Formel IIa gewinnt man durch Umsetzung von Triphenylphosphin mit geeigneten Alkylhalogeniden wie z.B. Methylbromid, Ethylbromid, Butylbromid, Isobutylbromid, Pentylbromid, Hexylbromid, Octyl-, Nonyl-, Decylbromid. Für diese Umsetzung sind auch die entsprechenden Chloride und Jodide geeignet. Man setzt in zahlreichen geeigneten Lösungsmitteln um wie Xylol, Toluol, Tetrahydrofuran, Dimethoxyethan, Diethoxyethan, Acetonitril, Dimethylsulfoxid, Dimethylformamid und N-Methylpyrrolidon bei Temperaturen von 80 - 150°C, bei höhersiedenden Halogeniden bei Normaldruck, bei niedersiedenden Halogeniden unter Überdruck. Die Phosphoniumsalze der Formel IIa sind in der Regel Feststoffe, die beim Abkühlen aus dem Lösungsmittel auskristallisierten. Sie werden abfiltriert und als Feststoffe in die Folgestufe eingesetzt. Eine Literaturübersicht betreffend Phosphoniumsalze gibt A. Maercker in Org. Reactions 14, 402ff. (1965). Vorteilhafter ist die Synthese der Phosphoniumsalze IIa aus Alkoholen der Formel VI, Triphenylphosphin und Halogenwasserstoff, wie in DE-A 10 46 046 beschrieben.

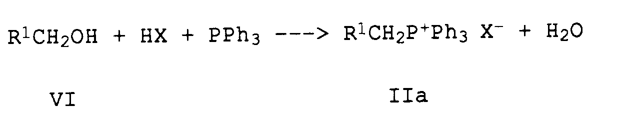

Als Lösungsmittel kann dabei der Alkohol VI dienen. Wenn es sich dabei um einen nicht oder nur gering mit Wasser mischbaren Alkohol handelt, kann die alkoholische Lösung von IIa in VI direkt in die Folgestufe eingesetzt werden. Zuvor muß jedoch das bei der Reaktion entstandene oder durch die Verwendung wäßriger Lösungen von HX eingebrachte Wasser durch Auskreisen entfernt werden.

Phosphoniumsalze der Formel IIb sind zugänglich aus cyclischen Ethern (K. Okuma, Bull. Chem. Soc. Jpn. 61, 4476 - 4478 (1988)) oder aus Bromalkoholen der Formel Vb (Herstellung siehe EP-A 266 544) und Triphenylphosphin (siehe z.B. M. Horiike et al. Agric. Biol. Chem. 44, (2) 257 - 261 (1980), M. Schlosser et al. Tetrahedron Lett. 26, 307 (1985)). 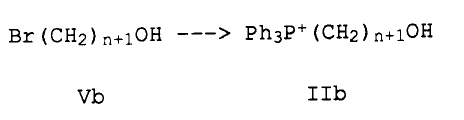
Geeignete Lösungsmittel für diese Umsetzung sind z.B. Methanol, Ethanol, Acetonitril, N-Methylpyrrolidon, Dimethylformamid, Xylol, Toluol, Chlorbenzol. Die Reaktionstemperaturen liegen zwischen 80 und 160°C. Als Lösungsmittel bevorzugt sind Alkohole, die nicht oder nur gering mit Wasser mischbar sind. Die Phosphoniumsalze IIb müssen dann nicht isoliert werden. Die Rohlösung von IIb kann direkt in die Folgestufe eingesetzt werden.

Wittig-Olefinierung, Herstellung der Alkenole I
Die Phosphoniumsalze der Formeln IIa und IIb werden in einem Alkohol gelöst oder suspendiert, z.B.: Methanol, Ethanol, 1-Propanol, 2-Propanol, n-Butanol, Isobutanol, n-Pentanol, 2-Pentanol, 3-Pentanol, 1-Hexanol, 2-Hexanol, 3-Hexanol, 1-Heptanol , 2-Heptanol, 1-Octanol, 1-Nonanol, 3-Heptanol, 2-Octanol, 2-Nona-nol, 1-Decanol, 2-Methyl-2-butanol und 2-Ethylhexanol.

Vorteilhafter ist es, wenn das Phosphoniumsalz IIa oder IIb bereits in einem geeigneten Alkohol wie oben beschrieben hergestellt worden ist, so daß eine Isolierung des Salzes entfällt.

Die Phosphoniumsalze IIa und IIb werden in der alkoholischen Lösung oder Suspension mittels einer Base in die entsprechenden Ylide überführt und anschließend mit den Aldehyden der Formel IIIa/IIIb oder IV zu den Alkenolen I umgesetzt.

Die Herstellung des Aldehyds der Formel IIIa (im Gleichgewicht mit seinem Halbacetal IIIb) 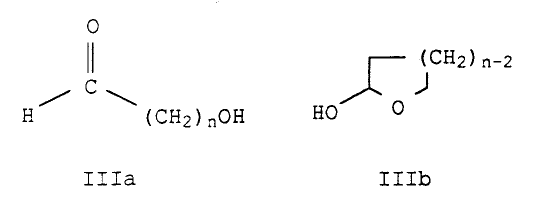
für n = 4 ist beschrieben in Organic Synthesis Coll. Vol. 3, S. 470. Der entsprechende Aldehyd mit n = 3 kann analog hergestellt werden. (Siehe auch G. Ohloff et al., Helv. Chim. Acta 60, 1161 (1977), R. Paul und S. Tchelitcheff, Bull. Soc. Chim. Fr., (5), 1, 971 (1934)).

Die oben beschriebene erfindungsgemäße Wittig-Olefinierung in Alkoholen, die nicht vollständig mit Wasser mischbar sind, führt wie andere literaturbekannte Verfahren zu Z/E-Isomerengemischen (siehe z.B. C. Szantay et al. Acta Chim. Hung. 125, 797 - 820 (1988)). Der Anteil der Z-Isomeren liegt zwischen 75 und 90%.

Die Verwendung von Alkoholen als Lösungsmittel bei Wittig-Reaktionen hat den Vorteil, daß die Reaktion wegen der guten Lösungseigenschaften hinsichtlich der Phosphoniumsalze konzentrierter gefahren werden kann, so daß die Raum/Zeit-Ausbeuten wesentlich steigen.

Bei der Verwendung von Alkoholen, die nicht vollständig oder nur wenig mit Wassser mischbar sind, entsteht ein weiterer Vorteil dadurch, daß die bei der wäßrigen Aufarbeitung entstehenden Abwässer nur einen geringen Anteil an organischen Verunreinigungen enthalten und über eine übliche Kläranlage entsorgt werden können.

Damit steht jetzt ein industriell verwertbares und ökonomisch wie ökologisch günstiges Verfahren zur Herstellung von Alkenolen zur Verfügung.

Alkenole der Formel I haben z.T. als Pheromone Bedeutung und können somit zur Schädlingsbekämpfung eingesetzt werden. Z.B.: Z5-Decenol, Z5-Dodecenol, Z7-Dodecenol, Z8-Dodecenol, Z9-Dodecenol, Z7-Tetradecenol, Z8-Tetradecenol, Z9-Tetradecenol, Z11-Tetradecenol, Z9-Hexadecenol, Z11-Hexadecenol, Z13-Octadecenol.

Durch Isomerisierung der Z-Alkenole sind die entsprechende E-Alkenole zugänglich (P.E. Sonnet, Tetrahedron 36, 557 - 604 (1980). E-Alkenole und Z-Alkenole können mit Acetylierungsmitteln (z.B. Acetanhydrid) zu den entsprechenden Acetaten umgesetzt werden. Ebenso wie die oben aufgeführten Z-Alkenole sind auch E-Alkenole und zahlreiche Acetate von Z- und E-Alkenolen als Pheromone bekannt und können im Pflanzenschutz Verwendung finden.

### Herstellungsbeispiele

### Beispiel 1

### 4-Tridecenol

Eine Mischung aus 576 g (4,0 Mol) 1-Nonanol, 262 g (1,0 Mol) Triphenylphosphin und 173,4 g (1,1 Mol) 47 %ige Bromwasserstoffsäure wird solange auf 100 - 120°C erhitzt bis ca. 112 ml Wasser ausgekreist sind. Anschließend rührt der Ansatz 6 Std. bei 140°C. Nach Abkühlen auf 25°C werden 99 g (1,8 Mol) Natriummethylat zugegeben, wobei die Reaktionstemperatur auf ca. 45°C ansteigt. Anschließend rührt die Reaktionsmischung 0,5 Std. bei 25°C. Bei 62°C werden 110 g (1,0 Mol) 80 %iges 2-Hydroxytetrahydrofuran zugegeben, wobei die Temperatur auf 90°C steigt. Man rührt 2 Std. bei 80°C nach und gibt die Reaktionsmischung auf 300 ml Wasser. Die organische Phase wird isoliert und nach Abdampfen der flüchtigen Bestandteile destilliert. Bei 120°/0,1 mbar destillierten 115 g (58 %) 4-Tridecenol, Isomerenverhältnis E:Z = 14:86.

### Beispiel 2

### 5-Decenol

Eine Mischung von 660 g (7,5 Mol) 1-Pentanol, 362 g (1,38 Mol) Triphenylphosphin und 262 g (1,55 Mol) 47 %ige Bromwasserstoffsäure werden solange auf 115 - 150°C erhitzt bis ca. 200ml Wasser ausgekreist sind. Anschließend rührt der Ansatz 6 Std. am Rückfluß. Nach Abkühlen auf 20°C werden 446 g (2,43 Mol) 30 %ige Natriummethylatlösung in Methanol zugegeben. Nachdem die Reaktionsmischung 0,5 Std. bei 20°C gerührt hat, werden bei 65°C und 1000 - 60 mbar ca. 390 ml Methanol abdestilliert. Bei 65°C werden anschließend 142 g (1,39 Mol) 2-Hydroxytetrahydropyran zugetropft. Man rührt 2 Std. bei 80°C nach und gibt den Ansatz auf 300 ml Wasser. Die organische Phase wird isoliert, von flüchtigen Anteilen befreit und destilliert. Man erhält 179 g (83 %) 5-Decenol Siedepunkt 77°/0,02 mbar, Isomerenverhältnis E:Z = 19:81.

### Beispiel 3

### 5-Decenol

Ein Gemisch von 453 kg (1,73 kMol) Triphenylphosphin, 697 kg (7,92 kMol) 1-Pentanol und 328 kg (1,90 kMol) 47 %ige HBr wird zum Rückfluß erwärmt. Nach Auskreisen von ca. 200 kg Wasser wird 6 h bei Rückfluß nachgerührt. Nach Abkühlen auf 20 - 25°C werden 171 kg (3,11 kMol) Natriummethylat portionsweise zugegeben. Man rührt 30 Min bei 20 - 25°C nach, heizt auf 65°C auf, läßt 177 kg (1,73 kMol 2-Hydroxytetrahydropyran bei 80°C zulaufen und rührt zwei Stunden bei 30 - 80°C nach. Man kühlt ab auf Raumtemperatur und läßt 300 l Wasser zulaufen. Die organische Phase wird destilliert. 216 kg 5-Decenol (80,0 %). Man erhält Isomerenverhältnis E:Z = 15:85.

### Beispiel 4

### 8-Dodecenol

Eine Mischung aus 335,4 g (1,28 Mol) Triphenylphosphin, 266,6 g (1,0 Mol) 78 %iges 8-Bromoctan-1-ol und 450 ml 1-Pentanol werden 5 Std. bei 110°C gerührt. Nach Abkühlen auf 20°C werden 140 g (2,6 Mol) Natriummethylat zugegeben. Dabei steigt die Temperaturauf 60°C an. Man rührt die Reaktionsmischung 2 Std. und tropft 92,3 g (1,28 Mol) Butyraldehyd zu. Nachdem 12 Std. bei 20°C nachgerührt wurde, gibt man den Ansatz auf 350 ml Wasser und isoliert die organische Phase. Destillation ergibt 89 g (48 %) 8-Dodecenol, Sdp.: 98 - 100°/1 mbar.

### Beispiel 5

### 8-Dodecenol

Eine Lösung von 335,4 g (1,28 Mol) Triphenylphosphin und 240,5 g (1,0 Mol) 8-Bromoctanol (86,9 %ig) in 400 ml 2-Butanol wird 8 h zum Rückfluß erwärmt. Man läßt abkühlen und gibt 140 g (2,6 Mol) Natriummethylat portionsweise zu. Dabei hält man die Reaktionstemperatur durch Kühlen unter 40°C. Man läßt eine Stunde nachrühren und tropft bis < 20°C 92,3 g (1,28 Mol) n-Butyraldehyd zu. Nach 2 h Nachrühren bei Raumtemperatur gibt man 350 ml Wasser zu. Destillaton der organischen Phase ergibt 117,4 g 8-Dodecenol (64 % Ausbeute). E:Z = 16:84.

## Patentansprüche

1. Verfahren zur Herstellung von Alkenolen der Formel I
R¹-CH=CH(CH₂)ₙOH I
in der R¹ für Wasserstoff oder einen Kohlenwasserstofferest steht und der Index n eine ganze Zahl von 3 bis 15 bedeutet,
a) durch Umsetzung eines Phosphoniumsalzes der Formel IIa
R¹-CH₂-P⁺(C₆H₅)₃ X⁻ IIa
in der X für Chlor, Brom und Jod steht, mit einem Aldehyd der Formel IIIa oder seinem Halbacetal der Formel IIIb 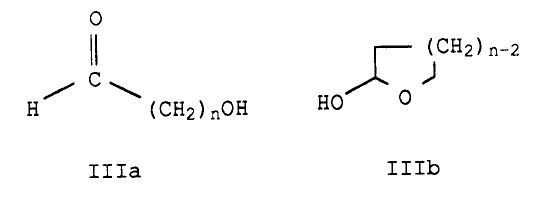 oder
b) durch Umsetzung eines Phosphoniumsalzes der Formel IIb
(C₆H₅)₃P⁺(CH₂)ₙ₊₁OH X⁻ IIb
mit einem Aldehyd der Formel IV
R¹CHO IV
in einem Lösungsmittel in Gegenwart einer Base, dadurch gekennzeichnet, daß man als Base das Alkalimetallsalz eines Alkohols und als Lösungsmittel einen Alkohol verwendet.

2. Verfahren zur Herstellung von Alkenolen der Formel I gemäß Anspruch 1, durch Umsetzung eines Alkylhalogenids der allgemeinen Formel Va bzw. Vb 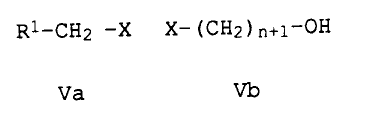 mit Triphenylphosphin und anschließende Kondensation der gebildeten Phosphoniumsalze der Formeln IIa bzw. IIb gemäß Anspruch 1 mit einem Aldehyd der Formel IIIa oder seinem Halbacetal der Formel IIIb bzw. mit einem Aldehyd der Formel IV gemäß Anspruch 1 in Gegenwart einer Base, dadurch gekennzeichnet, daß man diese Reaktionsfolge ohne Isolierung der Zwischenstufen in einem Alkohol als Lösungsmittel durchführt.

3. Verfahren zur Herstellung von Alkenolen der Formel I gemäß Anspruch 1, durch Umsetzung eines Alkohols der allgemeinen Formel VI
R¹-CH₂-OH VI
mit einer Halogenwasserstoffsäure zum entsprechenden Alkylhalogenid der Formel Va gemäß Anspruch 2, anschließender Umsetzung von Va mit Triphenylphosphin zum Phosphoniumsalz der Formel IIa gemäß Anspruch 1 und Kondensation des Phosphoniumsalzes der Formel IIa mit einem Aldehyd der Formel IIIa oder seinem Halbacetal der Formel IIIb gemäß Anspruch 1 in Gegenwart einer Base, dadurch gekennzeichnet, daß man diese Reaktionsfolge ohne Isolierung der Zwischenstufen im Alkohol VI als Lösungsmittel durchführt.

4. Verfahren zur Herstellung von 5-Decen-1-ol, durch Umsetzung von n-Pentanol mit einer Halogenwasserstoffsäure zum entsprechenden Pentylhalogenid, anschließender Umsetzung des Pentylhalogenids mit Triphenylphosphin zum entsprechenden Pentyl-triphenylphosphoniumsalz und Kondensation des Phosphoniumsalzes mit 5-Hydroxypentanal oder 2-Hydroxytetrahydropyran in Gegenwart einer Base, dadurch gekennzeichnet, daß man diese Reaktionsfolge ohne Isolierung der Zwischenstufen in n-Pentanol als Lösungsmittel durchführt.

5. Verfahren zur Herstellung von 4-Tridecen-1-ol, durch Umsetzung von n-Nonanol mit einer Halogenwasserstoffsäure zum entsprechenden Nonylhalogenid, anschließender Umsetzung des Nonylhalogenids mit Triphenylphosphin zum entsprechenden Nonyl-triphenylphosphoniumsalz und Kondensation des Phosphoniumsalzes mit 4-Hydroxybutanal oder 2-Hydroxytetrahydrofuran in Gegenwart einer Base, dadurch gekennzeichnet, daß man diese Reaktionsfolge ohne Isolierung der Zwischenstufen in n-Nonanol als Lösungsmittel durchführt.

6. Verfahren zur Herstellung von 8-Dodecen-1-ol durch Umsetzung von 8-Bromoctan-1-ol mit Triphenylphosphin und anschließende Kondensation des gebildeten Phosphoniumsalzes mit einem Aldehyd der Formel IV oder einem Aldehyd der Formel IIIa oder seinem Halbacetal der Formel IIIb in Gegenwart einer Base, dadurch gekennzeichnet, daß man diese Reaktionsfolge ohne Isolierung der Zwischenstufen in einem Alkohol als Lösungsmittel durchführt.

7. Verfahren zur Herstellung von 9-Dodecen-1-ol durch Umsetzung von 9-Bromoctan-1-ol mit Triphenylphosphin und anschließende Kondensation des gebildeten Phosphoniumsalzes mit einem Aldehyd der Formel IV oder einem Aldehyd der Formel IIIa oder seinem Halbacetal der Formel IIIb in Gegenwart einer Base, dadurch gekennzeichnet, daß man diese Reaktionsfolge ohne Isolierung der Zwischenstufen in einem Alkohol als Lösungsmittel durchführt.
